# EUROPÄISCHE PATENTSCHRIFT

(11) **EP 3 620 200 B1**
(45) Veröffentlichungstag und Bekanntmachung des Hinweises auf die Patenterteilung: **26.07.2023**
(21) Anmeldenummer: 19205810.5
(22) Anmeldetag: 05.06.2015
(51) Int. Cl.: A61M 25/00, B29C 65/16, B29C 65/00, B29L 31/00

(54) **KATHETER**
CATHETER
CATHÉTER

(30) Priorität: 14.08.2014 DE 102014011948
(43) Veröffentlichungstag der Anmeldung: 11.03.2020
(62) Teilanmeldung aus: 15170754.4
(73) Patentinhaber: BIOTRONIK AG, 8180 Bülach (CH)
(72) Erfinder: Westhoff, Felix, 8057 Zürich (DE); Schäfer, Tobias, 78176 Blumberg-Fützen (DE)
(74) Vertreter: Biotronik Corporate Services SE

(56) Entgegenhaltungen:
- EP-A1- 0 227 230
- US-A1- 2003 163 082
- US-A1- 2004 004 311
- US-A1- 2005 059 958
- US-A1- 2007 078 439

## Beschreibung

Dier vorliegende Erfindung betrifft Katheter.

Katheter weisen im Bereich ihres Schafts Röhrchen oder Schläuche verschiedenen Durchmessers auf, die in den jeweiligen zu behandelnden Körperhohlraum eingeführt werden können. Sogenannte Ballonkatheter werden vor allem in der Angioplastie zur Erweiterung oder Wiedereröffnung eines Gefäßes eingesetzt (perkutane transluminale koronare Angioplastie - PTCA, auch percutaneous coronary intervention oder perkutane Koronarintervention - PCI). Ein solcher Ballonkatheter besitzt einen Schaft, der in einem vorgegebenen Bereich einen zunächst nicht dilatierten Ballon aufweist. Bei der Behandlung wird zuerst ein Führungsdraht in das zu behandelnde Gefäß eingeschoben. Anschließend wird der Schaft des Ballonkatheters entlang des Führungsdrahts bis zu der zu behandelnden Stelle des Gefäßes vorgeschoben, so dass der Ballon im Bereich der zu behandelnden Stelle des Gefäßes platziert ist, die z.B. eine Stenose aufweist. Danach wird der Ballon dilatiert, d.h. entfaltet und/oder aufgedehnt, so dass die zu behandelnde Stelle wiedereröffnet oder erweitert wird. Schließlich wird der Ballon entleert und entlang des Führungsdrahts wieder aus dem Gefäß entfernt. Gleichzeitig oder anschließend wird auch der Führungsdraht aus dem Gefäß zurückgezogen. Derartige Ballonkatheter können zusätzlich auch dazu verwendet werden, intraluminale Endoprothesen an eine zu behandelnde Stelle in einem Körperhohlraum einzubringen.

Ein derartiger Katheter besitzt neben seinem Schaft in der Regel an seinem proximalen Ende ein Anschlussstück, welches zur Verbindung des Katheters mit der Kathetersteuerung und ggf. der Fluidversorgung dient. Das Anschlussstück ist mit dem Katheterschaft verbunden. Das distale Ende des Katheterschafts wird auch als Katheterspitze bezeichnet.

Insbesondere für die Anwendung in der Angioplastie werden Katheter mit mehreren, insbesondere zwei Lumen verwendet, wobei z.B. das erste Lumen zur Aufnahme des Führungsdrahts und das zweite Lumen zur Durchleitung einer Flüssigkeit oder eines Gases dient. Für Katheter, die in der Hämolyse eingesetzt werden, kann das erste Lumen für die Entnahme von Blut aus dem Körper des Patienten und das zweite Lumen für die Rückführung des gereinigten Bluts eingesetzt werden. Ein derartiger Mehrlumenkatheter wird in der Druckschrift WO 98/41277 A1 beschrieben.

Wie auch der Druckschrift WO 98/41277 A1 zu entnehmen ist, werden, wenn der Schaft zwei Lumen aufweist, grundsätzlich zwei Schafttypen unterschieden. Bei einem sogenannten Zweilumenschlauch werden das erste Lumen und das zweite Lumen nebeneinander, lediglich separiert durch eine Trennwand angeordnet. Beim koaxialen Schaftaufbau sind zwei zylindrische Lumen in hohlzylindrischen Elementen vorgesehen, wobei das kleinere hohlzylindrische Element (Innenschlauch) innerhalb des Lumens des größeren Hohlzylinders (Außenschlauch) angeordnet ist. Die Druckschrift US 6,248,092 B1 zeigt einen koaxialen Schaftaufbau. Auch die Druckschriften US 2004/004311 A1, US 2007/0078439 A1 und US 2003/0163082 A1 beschreiben mehrlumige Katheter.

Bei einem Zweilumenschlauch muss für die Anwendung im Bereich PTCA an dem Schaft ein Innenschlauch für das Ballonsegment und in vielen Fällen eine Führungsdrahtlumenverlängerung angebracht werden, um eine Durchgängigkeit des Führungsdrahtes durch den gesamten Katheter (vom Anschlussstück bis zur Katheterspitze) zu ermöglichen. In den Druckschriften EP 0 227 230 A1 und US 2005/0059958A1 werden Katheter mit einem Anschlussstück beschrieben.

Das Zweilumendesign hat zudem den Nachteil, dass in dem Bereich, in dem die Verlängerung an dem Schaft angebracht ist, kleinere Einschnürungen und Verhärtungen des Führungsdrahtlumens auftreten, welche die Push- und Trackability beeinflussen können. Zudem ist in diesem Bereich die Gefahr einer undichten Stelle gegeben. Da die Materialeigenschaften des Schafts alle Anforderungen des Systems erfüllen müssen, kann hierdurch oft nur ein Kompromiss zwischen Berstfestigkeit, FD-Reibung und Flexibilität hergestellt werden.

Der herkömmliche koaxiale Schaftaufbau zeichnet sich in nachteiliger Weise dadurch aus, dass der Durchmesser des Schaftprofils vergleichsweise groß ist. Zudem verschlechtern sich bauartbedingt die Push-Eigenschaften an der Katheterspitze.

Das konventionelle Koaxialdesign besitzt weiter den Nachteil, dass bei gleichen Abmessungen eine höhere Deflationszeit durch höhere Fluidreibung im Vergleich zum Zweilumendesign erreicht wird. Weiter kann es, da über einen Großteil des Schafts keine Verbindung von Innen- und Außenschlauch existiert, unter Belastung zu Relativbewegungen der ineinander verschachtelten zylindrischen Elemente kommen, welche die Track-/ Pusheigenschaften des Katheters negativ beeinflussen können.

Die Aufgabe besteht daher darin, die oben diskutierten Eigenschaften des Katheters zu verbessern.

Es wird ein Katheter mit einem Katheterschaft gemäß Anspruch 1 beschrieben.

Der Katheterschaft weist einen ersten Schlauch, welcher ein erstes Lumen ausbildet, und einen zweiten Schlauch auf, welcher ein zweites Lumen ausbildet und zumindest teilweise innerhalb des ersten Schlauchs angeordnet ist. Hierbei ist der zweite Schlauch zumindest abschnittsweise mit seiner äußeren Oberfläche (Mantelfläche) mit der Innenfläche des ersten Schlauch verschweißt. Hierfür muss der Innendurchmesser des ersten Schlauchs größer sein als der Außendurchmesser des zweiten Schlauchs. Vorzugsweise bilden der Außendurchmesser des zweiten Schlauchs und der Innendurchmesser des ersten Schlauchs ein Verhältnis, das zwischen 0,4 und 0,95, besonders bevorzugt zwischen 0,6 und 0,9, liegt. D.h. der Außendurchmesser des zweiten, inneren Schlauchs beträgt mindestens das 0,4fache, bevorzugt das 0,6fache, des Innendurchmessers des ersten, äußeren Schlauchs und maximal das 0,95fache, bevorzugt das 0,9fache, des Innendurchmessers des ersten, äußeren Schlauchs.

Das (innere) Lumen des ersten Schlauchs bildet vorzugsweise das Inflationslumen, während das (innere) Lumen des zweiten Schlauchs, d.h. das kleinere Lumen, vorzugsweise das Führungsdrahtlumen darstellt.

Durch einen derartigen Aufbau eines Katheterschafts vereinfacht sich die Systemmontage, so dass die Kosten für die Herstellung eines Katheters reduziert und weniger Ausschuss produziert wird. Durch die Verwendung zweier ineinander angeordneter, aneinander befestigter Schläuche wird zudem die FD-Gängigkeit verbessert, da das Führungsdrahtlumen komplett homogen und rund ausgebildet ist. Im Vergleich zum herkömmlichen koaxialen Schaftaufbau ohne Verbindung von Innen- und Außenschlauch kann die Deflationszeit verringert und ein erhöhter Push erreicht werden.

Bei dem Katheterschaft sind der erste Schlauch und der zweite Schlauch derart ineinander angeordnet, dass die Längsachsen der Schläuche, welche vorzugsweise jeweils einen kreisförmigen Querschnitt aufweisen, parallel zueinander verlaufen. Andere Querschnittsformen als die Kreisform für den ersten Schlauch und/oder den zweiten Schlauch sind ebenfalls denkbar (z.B. rechteckiger Querschnitt).

In einem besonders bevorzugten Ausführungsbeispiel erstreckt sich die Schweißnaht parallel zur Längsachse des ersten Schlauchs und/oder des zweiten Schlauchs. Hierdurch werden Einschnürungen im Bereich des Schafts vermieden, so dass ebenfalls die Push- und Trackability-Eigenschaften verbessert werden.

Es ist weiter von Vorteil, wenn der erste Schlauch zumindest im Bereich seiner inneren Oberfläche und/oder der zweite Schlauch zumindest im Bereich seiner äußeren Oberfläche (d.h. an seiner Mantelfläche) ein laseraktives Material aufweist. Ein derartiges laseraktives Material erwärmt sich bei Laserbestrahlung mit einer bestimmten Wellenlänge. Über diese Bereiche mit laseraktiven Materialien lassen sich dann der erste und der zweite Schlauch leicht verschweißen, so dass die Systemmontage weiter vereinfacht wird. Die verwendete Wellenlänge des Lasers ist materialabhängig und wird vorteilhafterweise entsprechend ausgewählt. Weiter lassen sich der erste Schlauch und der zweite Schlauch so verbinden, dass keine Deformation der Oberfläche der Schläuche entsteht, welche die Eigenschaften des Katheterschafts negativ beeinflussen kann.

Nichtlaseraktive Materialien können durch Wärmestrahlung (z.B. mittels Schweißbacken, Heißluft oder Laser) verbunden werden.

Der Katheterschaft kann weiter dadurch verbessert werden, dass der erste Schlauch eine erste chemische Zusammensetzung und der zweite Schlauch eine zweite chemische Zusammensetzung aufweist, wobei die zweite chemische Zusammensetzung von der ersten chemischen Zusammensetzung verschieden ist. In einer vorteilhaften Ausgestaltung der Erfindung besteht der zweite Schlauch aus einem Polyamid, beispielsweise PA12. Polyamid gewährleistet eine geringe Reibung zwischen Führungsdraht und Schlauch. Der erste Schlauch besteht in dieser Ausgestaltung vorteilhafterweise aus einem thermoplastischen Copolyamid, beispielsweise PEBAX, wodurch insgesamt eine hohe Flexibilität des Kathetersystems erreicht wird. Durch diese erfindungsgemäße Lösung können die Materialien der Schläuche für den jeweiligen Einsatzzweck gemäß der jeweiligen Anforderungen (z.B. hinsichtlich Berstdruck Flexibilität, Beschichtung, FD-Reibung) optimiert und somit auch die Eigenschaften des Gesamtsystems verbessert werden.

Vorteilhafterweise besteht der erste und/oder der zweite Schlauch aus mindestens einem Material ausgewählt aus der Gruppe enthaltend Polyamide, insbesondere PA12, thermoplastische Elastomere, insbesondere thermoplastische Copolyamide wie PEBAX 7033, PEBAX 7233, Grilamid L25 und Vestamid L2101.

Die obige Aufgabe wird weiter durch einen Katheter mit einem Katheterschaft mit den oben angegebenen neuen Merkmalen gelöst. Hierdurch weist der erfindungsgemäße Katheter die oben angegebenen Vorteile auf.

Der Schaft des erfindungsgemäßen Katheters ist mit einem Anschlussstück verbunden, welches mindestens einen konischen Abschnitt aufweist, auf dem das proximale Ende des ersten Schlauchs oder des zweiten Schlauchs angeordnet ist. Der Konus ist hierbei derart beschaffen, dass der äußere Querschnitt des Abschnitts in proximaler Richtung zunimmt. Weiter weist der konische Abschnitt vorzugsweise eine innenliegende durchgehende Öffnung auf. Der Vorteil dieser Lösung besteht darin, dass der Schaftschlauch beim Aufstecken auf das Anschlussstück automatisch proximal abgedichtet wird. Hierdurch kann die Montage des Katheters weiter vereinfacht und eine besonders gute Abdichtung im Bereich des Abschlussstücks erreicht werden. Der konische Abschnitt ist vorzugsweise innerhalb eines im Wesentlichen hohlzylinderförmigen Körpers des Anschlussstücks an dessen proximalem Ende angeordnet.

Durch den Konus wird das Lumen des Führungsdrahts, wenn der Schlauch des Führungsdrahts auf dem konischen Abschnitt des Anschlussstücks angeordnet ist, aufgeweitet. Neben dem dichten Abschluss wird ein sanfter Übergang von dem Schaftschlauch zum Anschlussstück realisiert, so dass eine Kante, an der der Führungsdraht hängen bleiben könnte, vermieden wird.

In einem weiteren bevorzugten Ausführungsbeispiel kann das Anschlussstück mehrteilig ausgebildet sein, nämlich mit mindestens einem distalen Element und einem proximalen Element mit dem konischen Abschnitt, wobei das distale Element mit dem proximalen Element vorzugsweise mittels einer Schnappverbindung, einer Schweißverbindung oder einer anderen vorzugsweise nicht lösbaren Verbindung miteinander verbindbar sind. Durch die mehrteilige Ausbildung des Anschlussstücks kann eine sichere Befestigung des Katheterschafts erzielt werden, so dass auf eine Verklebung mit dem Katheterschaft verzichtet werden kann. Zusätzlich kann das distale Element an seinem distalen Ende eine Knickschutz-Einrichtung aufweisen. Diese besteht vorzugsweise aus ringförmigen Elementen, welche mittels in Längsrichtung (axiale Richtung) verlaufenden Stegen miteinander verbunden sind. Die Knickschutz-Einrichtung ist dabei derart dimensioniert, dass sie den ersten Schlauch des Katheterschafts nach der Montage des Anschlussstücks außen umgibt.

In einer bevorzugten Ausführungsform der Ausgestaltung als mehrteiliges Anschlussstück wird das distale Element in einem Zweikomponentenspritzgussverfahren hergestellt. Die Knickschutz-Einrichtung besteht dabei aus einem weicheren und elastischeren Material als der Rest des distalen Elements. Durch die Verwendung eines weicheren Materials wird der Knickschutz effizienter und kann mit optionalen Dichtungen aus demselben Material besser abgedichtet werden. Die Haltekraft der Katheterschläuche am Anschlussstück wird dadurch ebenfalls erhöht. Auch für das proximale Element kann die Verwendung verschiedener Materialien vorteilhaft wirken. Wird hier für das konische, distale Ende des proximalen Elements ein weicheres und elastischeres Material verwendet, wird die Dichtigkeit verbessert und über die elastischen Eigenschaften eine Federwirkung erzielt. Diese Federwirkung hält den Druck bei einer Schnappverbindung über eine lange Zeit aufrecht.

Als weicheres Material für die Knickschutz-Einrichtung wird vorzugsweise ein Polyether-Block-Amid (PEBA oder TPE-A, wie beispielsweise erhältlich unter den Handelsnamen PEBAX ^{®}, Vestamid E^{®}, Grilflex ^{®}), ein thermoplastisches Elastomer (TPE), insbesondere ein Polyester-Elastomer (TPE-E, z.B. erhältlich unter dem Handelsnamen Hydrel ^{®}) eingesetzt. Als weiteres Material für die übrigen Elemente des Anschlussstücks wird bevorzugt ein amorphes Polyamid (z.B. erhältlich unter den Handelsnamen Grilamid TR ^{®}, Trogamid ^{®}), ein Polycarbonat (PC) (beispielsweise erhältlich unter der Bezeichnung Makrolon ^{®}) oder ein Polyethylenterephthalate (PET) verwendet.

Besonders bevorzugt kann insbesondere bei der einteiligen Ausführungsform des Anschlussstücks dieses zusätzlich an der Innenseite seines distalen Endes mit der Außenfläche des ersten Schlauchs verklebt sein, so dass ein Abrutschen des jeweiligen, auf dem konischen Abschnitt des Anschlussstücks angeordneten Schlauchs vom Konus verhindert wird.

Die oben beschriebenen Lösungen mit dem konischen Abschnitt des Anschlussstücks ermöglichen zudem eine effektive Automatisierung des Fügeprozesses von Schaft und Anschlussstück.

Für den Fall, dass gleichzeitig das Inflationslumen (z.B. erster Schlauch mit Lumen für ein Fluid, nicht für den Führungsdraht) durch die Ausweitung des Führungsdrahtlumens (z.B. zweiter Schlauch) verschlossen wird, kann ein zusätzlicher Schnitt am Inflationslumen Abhilfe schaffen. Dieser Schnitt könnte beispielsweise direkt beim Verbinden des Anschlussstücks mit dem proximalen Ende des Schafts erfolgen. Dafür wird im Inflationslumen eine Kunststoffklinge vorgesehen, welche den Schnitt bewirkt. Die Kunststoffklinge wird bevorzugt am Anschlussstück auf einer parallel zur Längsachse verlaufenden Nut oder Rippe angeordnet.

Es wird ferner ein Verfahren zur Herstellung eines Katheterschafts beschrieben, welches die folgenden Schritte beinhaltet:
- Bereitstellung eines ersten Schlauchs und eines zweiten Schlauchs,
- Anordnen des zweiten Schlauchs zumindest teilweise in dem ersten Schlauch und
- Verschweißen des zweiten Schlauchs zumindest abschnittsweise mit seiner äußeren Oberfläche mit der Innenfläche des ersten Schlauchs.

Hierbei werden der erste Schlauch und der zweite Schlauch derart ineinander angeordnet, dass die Längsachsen der beiden Schläuche, welche vorzugsweise jeweils einen kreisförmigen Querschnitt aufweisen, nach der Fertigstellung parallel zueinander verlaufen. Weiter erfolgt in einem besonders bevorzugten Ausführungsbeispiel das Verschweißen derart, dass sich die Schweißnaht parallel zur Längsachse des ersten Schlauchs und/oder des zweiten Schlauchs erstreckt.

Das Verfahren ist besonders einfach und kostengünstig durchführbar.

Es wird ferner ein Verfahren zur Herstellung eines Katheters beschrieben, bei dem die oben angegebenen Schritte zur Herstellung eines Katheterschafts durchgeführt werden sowie anschließend der zusätzliche Schritt, dass der Katheterschaft an seinem proximalen Ende mit einem einteiligen Anschlussstück oder einem mehrteiligen Anschlussstück mit mindestens einem distalen Element und einem proximalen Element verbunden wird.

In einem besonders bevorzugten Ausführungsbeispiel wird hierbei das proximale Ende des Katheterschafts in den vorzugsweise hohlzylindrischen Körper des einteiligen Anschlussstücks eingeführt. Besonders bevorzugt wird dabei der erste Schlauch oder der zweite Schlauch über einem oben beschriebenen konischen Abschnitt des Anschlussstücks angeordnet, so dass automatisch eine Abdichtung des Schlauchs erfolgt. Hierbei wird das Lumen des entsprechenden Schlauchs in diesem Bereich aufgeweitet. Nach dem Einführen und Verbinden des Katheterschafts mit dem Anschlussstück wird vorzugsweise das Anschlussstück an der Innenseite des distalen Endes seines Körpers mit der Außenfläche des ersten Schlauchs und/oder des zweiten Schlauchs verklebt.

Alternativ, bei der Verwendung eines mehrteiligen Anschlussstücks, wird das proximale Ende des Katherschafts in eine durchgehende Öffnung des distalen Elements eingefädelt, anschließend das proximale Element mit seinem konischen Abschnitt in das proximale Ende des Katheterschafts, vorzugsweise in das proximale Ende des zweiten Schlauchs, eingeführt und danach das distale Element in proximaler Richtung zum proximalen Element des Anschlussstücks verschoben und mit diesem, z.B. mittels einer Schnappverbindung, verbunden. Dies ist ein besonders einfaches und kostengünstiges Vorgehen zum Befestigen des Katheterschafts an dem Anschlussstück. Hierbei wird das proximale Ende des Katheterschafts beim Einfädeln in das distale Element auch durch eine durchgehende Öffnung der Knickschutz-Einrichtung hindurchgefädelt.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: einen Katheterschaft in einer Ansicht von der Seite,
- Fig. 2: den Katheterschaft gemäß Fig. 1 im Querschnitt und
- Fig. 3: einen Längsschnitt durch einen erfindungsgemäßen Katheter im Bereich eines ersten Ausführungsbeispiels eines Anschlussstücks,
- Fig. 4: ein zweites Ausführungsbeispiel eines Anschlussstücks für einen erfindungsgemäßen Katheter mit einem distalen Element und einem mit dem distalen Element verbundenen proximalen Element in einer perspektivischen Ansicht von der Seite,
- Fig. 5: das Ausführungsbeispiel des Anschlussstücks gemäß Fig. 4, wobei das distale Element und das proximale Element getrennt in einer perspektivischen Ansicht von hinten dargestellt sind,
- Fig. 6: eine weitere getrennte Darstellung des distalen Elements und des proximalen Elements des Ausführungsbeispiels eines Anschlussstücks gemäß Fig. 4 in einer perspektivischen Ansicht von der Seite,
- Fig. 7: das Ausführungsbeispiel des Anschlussstücks gemäß Fig. 4 mit verbundenem distalen Element und proximalen Element in einem Querschnitt,
- Fig. 8: ein drittes Ausführungsbeispiel eines Anschlussstücks für einen erfindungsgemäßen Katheter mit einem distalen Element und einem mit dem distalen Element verbundenen proximalen Element in einer perspektivischen Ansicht von der Seite,
- Fig. 9: das Ausführungsbeispiel eines Anschlussstücks gemäß Fig. 8 in einem Querschnitt,
- Fig. 10: der Querschnitt gemäß Fig. 9 des Ausführungsbeispiels eines Anschlussstücks gemäß Fig. 8, wobei das distale Element und das proximale Element separat dargestellt sind,
- Fig. 11-: eine weitere getrennte Darstellung des distalen Elements und des proximalen Elements des Ausführungsbeispiels gemäß Fig. 8 in einer Ansicht von der Seite,
- Fig. 12: eine weitere getrennte Darstellung des distalen Elements und des proximalen Elements des Ausführungsbeispiels gemäß Fig. 8 in einem Querschnitt,
- Fig. 13: das Ausführungsbeispiel eines Anschlussstücks gemäß Fig. 8 in einem weiteren Querschnitt,
- Fig. 14: eine weitere getrennte Darstellung des distalen Elements und des proximalen Elements des Ausführungsbeispiels gemäß Fig. 8 in einer perspektivischen Ansicht von der Seite und
- Fig. 15: eine weitere getrennte Darstellung des distalen Elements und des proximalen Elements des Ausführungsbeispiels gemäß Fig. 8 geschnitten, in einer perspektivischen Ansicht von der Seite.

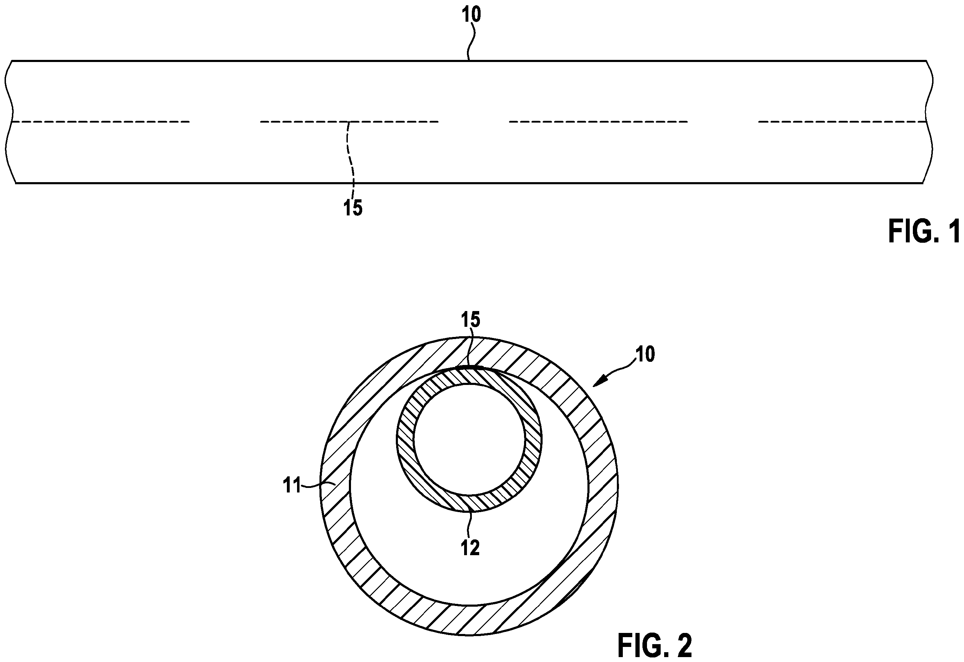

Die Figuren 1 und 2 zeigen einen Katheterschaft 10 mit einem ersten Schlauch 11 und einem zweiten Schlauch 12, wobei der erste Schlauch 11 und der zweite Schlauch 12 parallel zu ihrer Längsachse ineinander angeordnet sind. Im Bereich seiner äußeren Oberfläche (Mantelfläche) ist der zweite Schlauch 12 mit dem ersten Schlauch 11, d.h. insbesondere mit seiner Innenfläche, entlang einer Schweißnaht 15 verschweißt. Die Schweißnaht 15 verläuft parallel zur Längsachse des ersten Schlauchs 11 beziehungsweise des zweiten Schlauchs 12, wobei die Schweißnaht 15 sich entweder über die gesamte Länge des Katheterschafts erstreckt oder abschnittsweise, wie in Figur 1 gezeigt, verläuft.

Um eine gute Verschweißung zwischen dem ersten Schlauch 11 und dem zweiten Schlauch 12 zu erreichen, weist der erste Schlauch 11 in dem Bereich seiner Innenfläche und/oder der zweite Schlauch 12 im Bereich seiner äußeren Mantelfläche ein laseraktives Material auf. Der erste Schlauch 11 kann beispielsweise aus PEBAX und der zweite Schlauch 12 aus PA12 bestehen. Das laseraktive Material ist bevorzugt auf der Mantelfläche des zweiten Schlauchs 12 aufgebracht.

Der zweite Schlauch 12 kann beispielsweise zur Anordnung eines Führungsdrahts 23 dienen, während der erste Schlauch 11 das Inflationslumen ausbildet.

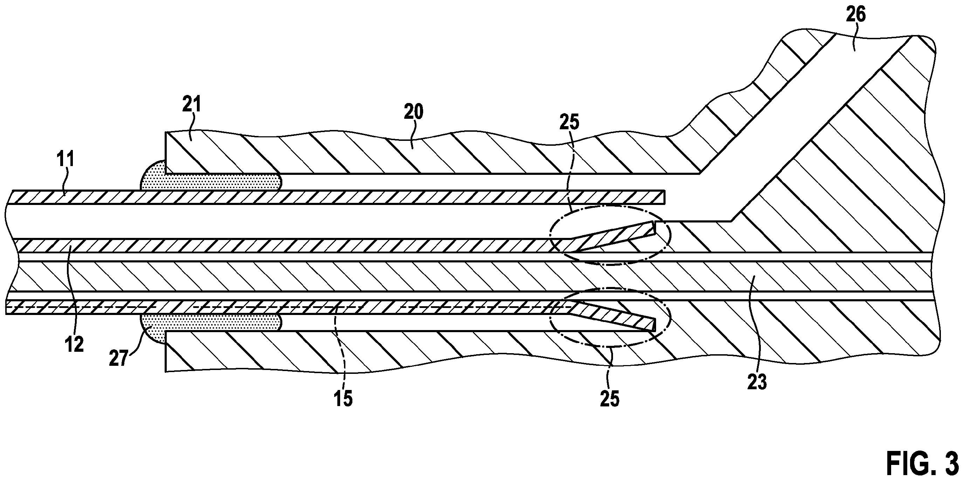

Der in Figur 3 gezeigt erfindungsgemäße Katheter besitzt ein Anschlussstück 20, welches mit dem proximalen Ende des Katheterschafts 10 verbunden ist. Der Körper des Anschlussstücks 20 ist an seinem distalen Ende 21 hohlzylinderförmig ausgebildet, so dass sowohl der erste Schlauch 11 als auch der zweite Schlauch 12 des Katheterschafts 10 in die Öffnung des Anschlussstücks 20 eingeführt werden können. An dem proximalen Ende des Anschlussstücks 20 ist zudem ein konischer Abschnitt 25 vorgesehen, auf den der zweite Schlauch 12 aufgeschoben wird, so dass dieser Schlauch 12 automatisch abgedichtet wird. Ferner weist das Anschlussstück 20 einen Kanal 26 auf, in den das Lumen des ersten Schlauchs 11 mündet und dieses mit einer Fluidquelle zur Inflation/Deflation eines Ballons verbindet.

Nachdem das proximale Ende des Katheterschafts 10 in das Anschlussstück 20 vollständig eingeführt wurde, wird im Bereich des distalen Endes 21 des Körpers des Anschlussstücks 20 die Außenfläche des ersten Schlauchs 11 und die Außenfläche des zweiten Schlauchs 12 mit der Innenfläche des Anschlussstücks mittels eines Klebstoffs 27 verklebt, so dass der erste Schlauch 11 nicht vom konischen Abschnitt 25 abrutscht.

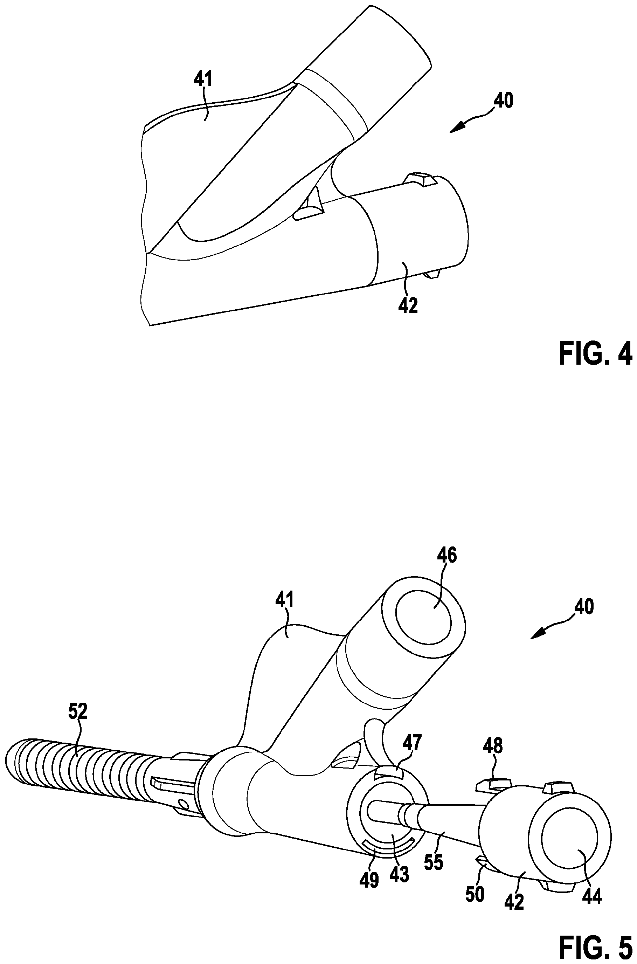

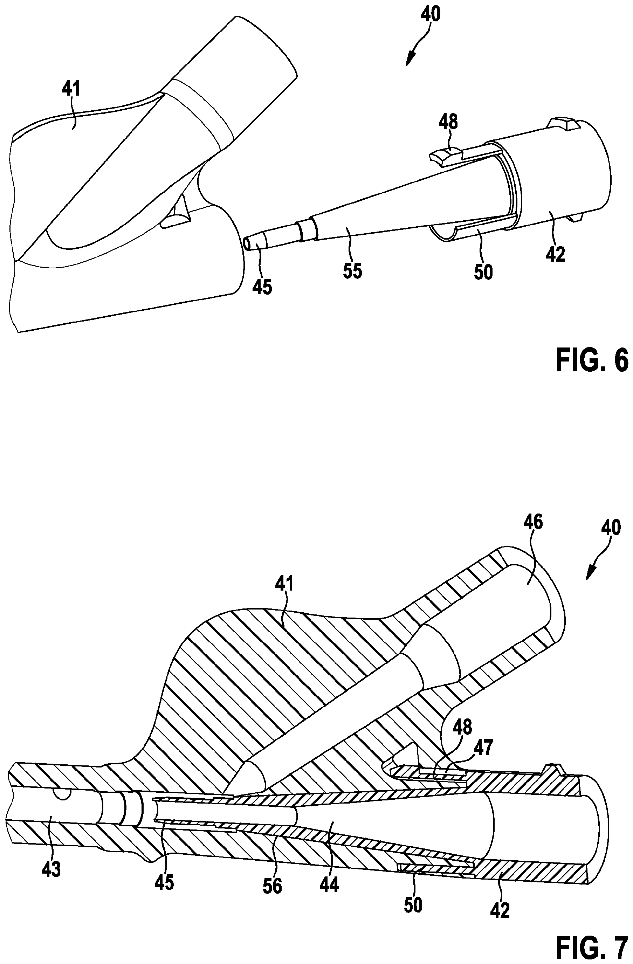

In den Figuren 4 bis 7 ist ein zweites Ausführungsbeispiel eines Anschlussstücks 40 für einen erfindungsgemäßen Katheter gezeigt, das zweiteilig mit einem distalen Element 41 und einem proximalen Element 42 ausgebildet ist. Beide Elemente 41, 42 sind im Wesentlichen hohlzylinderförmig gestaltet.

Das distale Element 41 weist eine durchgehende Öffnung 43 auf, in die das proximale Ende des Katheterschafts eingeführt werden kann. Entsprechend besitzt das proximale Element 42 eine durchgehende Öffnung 44 zur Anordnung des Führungsdrahts. An seinem distalen Ende 45 ist an der Außenseite (Mantel) des proximalen Elements 42 ein erster konischer Abschnitt vorgesehen. Weiter sieht das distale Element 41 einen schräg verlaufenden Kanal 46 zur Verbindung mit einer Fluidquelle zur Inflation/Deflation eines an den Katheterschaft angeschlossenen, nicht dargestellten Ballons vor. Der Kanal 46 erstreckt sich bis in die durchgehende Öffnung 43 des distalen Elements 41.

Das distale Element 41 besitzt weiter an seinem proximalen Ende ein schlitzförmiges Sackloch 47 mit einer Hinterschneidung zur Anordnung eines von dem proximalen Element 42 in distaler Richtung vorstehenden Stegs 48 mit einer hakenförmigen Verdickung an seinem distalen Ende. Weiter ist an dem proximalen Ende des distalen Elements 41 eine im Querschnitt gerundete Führungsnut 49 vorgesehen, welche beim Zusammenfügen des distalen Elements 41 mit dem proximalen Element 42 mit einem entsprechend geformten Steg 50 an dem distalen Ende des proximalen Elements 42 kooperiert.

Das distale Element 41 weist ferner an seinem distalen Ende ein Knickschutzelement 52 auf, das aus nebeneinander liegenden ringförmigen Elementen zusammengesetzt ist, welche mittels in Längsrichtung verlaufenden Stegen verbunden sind.

Weiter ist an dem proximalen Element 42 in einem mittigen Bereich in Bezug auf die Ausdehnung des Elements in Längsrichtung ein zweiter konischer Abschnitt 55 vorgesehen. Entsprechend weist das distale Element an seinem proximalen Ende im Bereich der Innenwand ebenfalls einen konischen Abschnitt 56 auf.

Zur Herstellung des erfindungsgemäßen Katheters mittels eines oben beschriebenen Katheterschafts und des in den Figuren 4 bis 7 dargestellten Anschlussstücks 40 wird zunächst das proximale Ende des Katheterschafts in die durchgehende Öffnung 43 des distalen Elements 41 hineingeschoben. Das Einschieben kann durch den Führungsdraht unterstützt werden. Danach sind die ringförmigen Elemente und Stege des Knickschutzelements 52 auf der Außenseite des ersten Schlauchs 11 angeordnet.

Anschließend wird das proximale Element 42 in das innere Lumen des zweiten Schlauchs 12 soweit eingeführt, dass der zweite konische Abschnitt 55 des proximalen Elements 42 aus dem zweiten Schlauch hervorschaut. Der erste konische Abschnitt an dem distalen Ende 45 des proximalen Elements 62 ist demgegenüber in dem zweiten Schlauch 12 angeordnet.

Danach wird das distale Element 41 in proximale Richtung auf dem Katheterschaft in Richtung proximales Element 42 verschoben, wobei der Steg 48 in das Sackloch 47 und der Steg 50 in die Nut 49 eingeführt wird. Am Ende des Verschiebewegs hintergreift die hakenförmige Verdickung des Stegs 48 die Hinterschneidung des Sacklochs 47 und bewirkt somit eine Befestigung des proximalen Elements 42 an dem distalen Element 41 in Form einer Schnappverbindung. Durch den ersten konischen Abschnitt 45 wird gleichzeitig der zweite Schlauch 12 abgedichtet. Weiter bewirkt ein Formschluss zwischen dem zweiten konischen Abschnitt 55 des proximalen Elements 42 und dem konischen Abschnitt 56 des distalen Elements 41 eine Abdichtung des Inflationslumens des ersten Schlauchs 11.

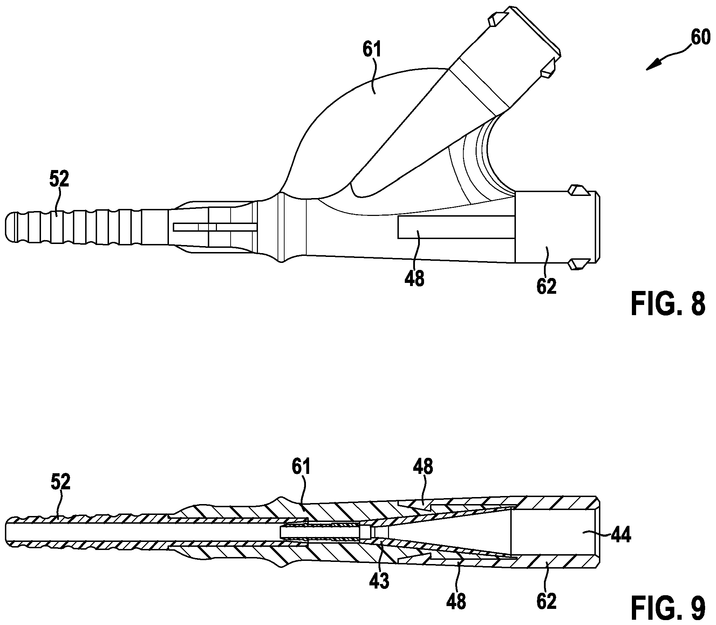

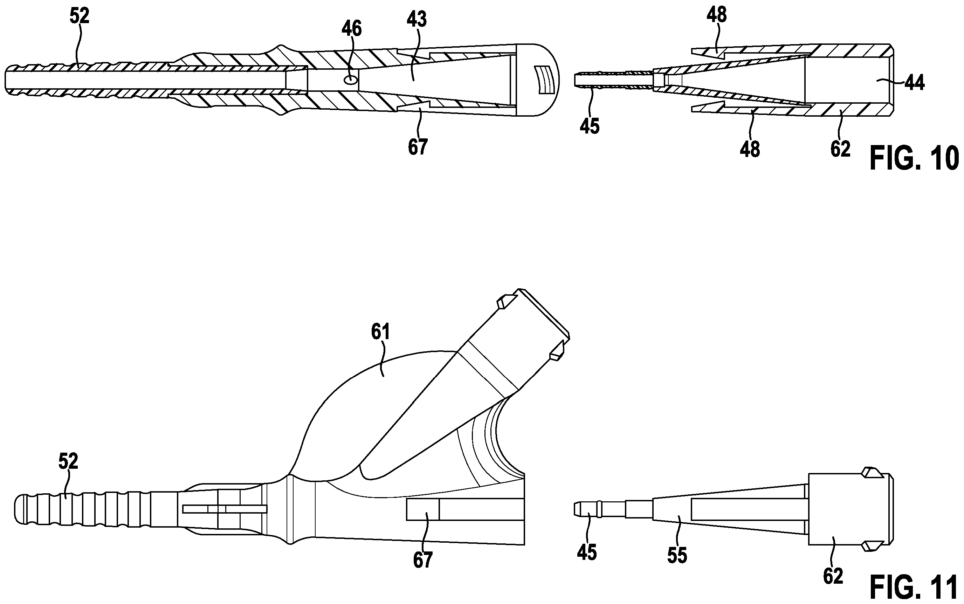

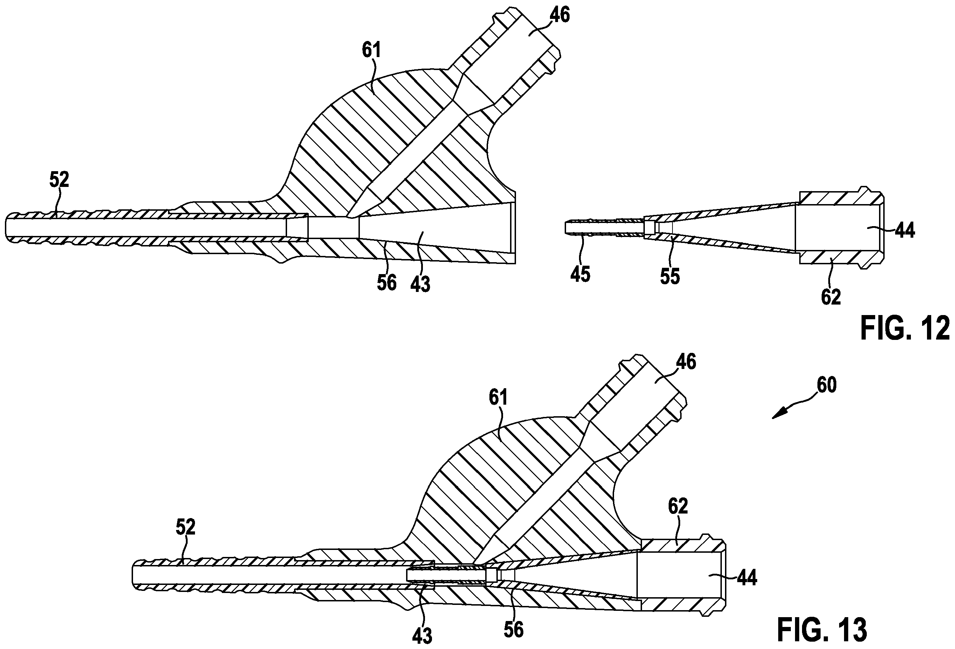

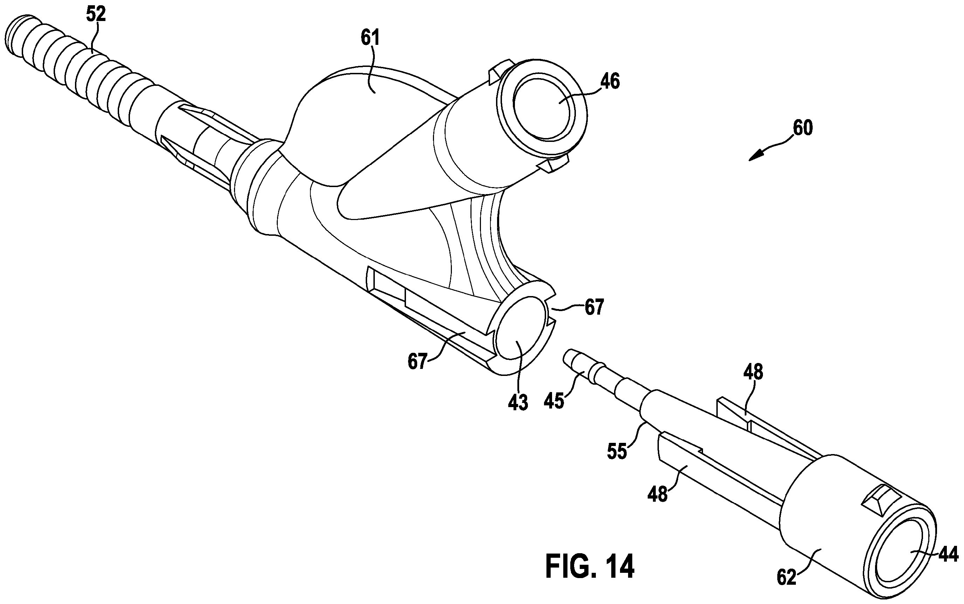

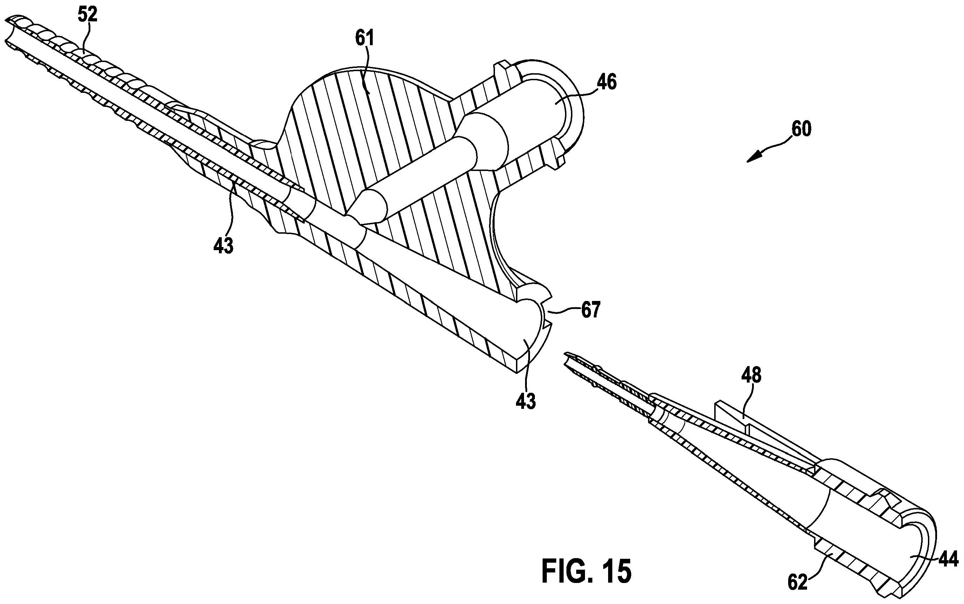

In den Figuren 8 bis 15 ist ein drittes Ausführungsbeispiel eines Anschlussstücks 60 für einen erfindungsgemäßen Katheter gezeigt, das zweiteilig mit einem distalen Element 61 und einem proximalen Element 62 ausgebildet ist. Beide Elemente 61, 62 sind im Wesentlichen analog zu dem zweiten Ausführungsbeispiel gemäß Fig. 4 bis 7 und hohlzylinderförmig gestaltet. Die Bezugszeichen der Elemente 61, 62, welche denen des zweiten Ausführungsbeispiels entsprechen, beziehen sich auf gleiche Bereiche des Anschlussstücks.

Bei dem dritten Ausführungsbeispiel eines Anschlussstücks erfolgt eine andere Befestigung des distalen Elements 61 an dem proximalen Element 62, nämlich mittels in distaler Richtung von dem proximalen Element 62 vorstehender zwei Stege 48 mit hakenförmiger Verdickung an seinem distalen Ende und entsprechend geformter Kerben (Ausnehmungen) 67, welche in der äußeren Oberfläche des distalen Elements 61 angebracht sind. Jede Kerbe weist eine Hinterschneidung auf, welche zur Anordnung der hakenförmigen Verdickung an dem distalen Ende des Stegs 48 dient. Hierdurch wird beim Befestigen des proximalen Elements ein Formschluss erzielt. Die Kerben 67 dienen gleichzeitig zur Führung der Stege 48. Daher ist bei dieser Ausführungsform kein zusätzlicher Führungssteg wie bei dem zweiten Ausführungsbeispiel erforderlich.

Weiter ist das distale Ende 45 des proximalen Elements 62 etwas anders geformt. Es weist mehrere nebeneinander liegende Abschnitte auf, die teilweise konisch ausgebildet sind.

Mit den in den Figuren 4 bis 15 dargestellten mehrteiligen Anschlussstücken 40 und 60 wird eine einfache Montage des Katheters erreicht, welche eine sichere und fluiddichte Verbindung zwischen Katheterschaft und Anschlussstück bewirkt.

### Bezugszeichenliste

- 10: Katheterschaft
- 11: erster Schlauch
- 12: zweiter Schlauch
- 15: Schweißnaht
- 20: Anschlussstück
- 21: distales Ende des Körpers des Anschlussstücks 20
- 23: Führungsdraht
- 25: konischer Abschnitt
- 26: Kanal
- 27: Klebstoff
- 40, 60: Anschlussstück
- 41, 61: distales Element
- 42, 62: proximales Element
- 43: durchgehende Öffnung
- 44: durchgehende Öffnung
- 45: distales Ende des proximalen Elements 42, 62
- 46: Kanal zur Inflation/Deflation
- 47: Sackloch
- 48: Steg
- 49: Nut
- 50: Steg
- 52: Knickschutz-Einrichtung
- 55: zweiter konischer Abschnitt
- 56: konischer Abschnitt
- 67: Kerbe

## Patentansprüche

1. Katheter mit einem Katheterschaft (10) aufweisend einen ersten Schlauch (11), welcher ein erstes Lumen ausbildet, und einen zweiten Schlauch (12), welcher ein zweites Lumen ausbildet und zumindest teilweise innerhalb des ersten Schlauchs (11) angeordnet ist, wobei der zweite Schlauch (12) zumindest abschnittsweise mit seiner äußeren Oberfläche mit der Innenfläche des ersten Schlauchs (11) verschweißt ist, **dadurch gekennzeichnet, dass** der Katheterschaft (10) mit einem Anschlussstück (20, 40) verbunden ist, welches mindestens einen konischen Abschnitt (25, 45) aufweist, auf dem das proximale Ende des zweiten Schlauchs (12) aufgeschoben ist.

2. Katheter nach Anspruch 1, **dadurch gekennzeichnet, dass** sich die Schweißnaht (15) parallel zur Längsachse des ersten Schlauchs (11) und/oder des zweiten Schlauchs (12) erstreckt.

3. Katheter nach einem der vorhergehenden Ansprüche, **dadurch gekennzeichnet, dass** der erste Schlauch (11) zumindest im Bereich seiner inneren Oberfläche und/oder der zweite Schlauch (12) zumindest im Bereich seiner äußeren Oberfläche ein laseraktives Material aufweist.

4. Katheter nach einem der vorhergehenden Ansprüche, **dadurch gekennzeichnet, dass** der erste Schlauch (11) eine erste chemische Zusammensetzung und der zweite Schlauch (12) eine zweite chemische Zusammensetzung aufweist, wobei die zweite chemische Zusammensetzung von der ersten chemischen Zusammensetzung verschieden ist.

5. Katheter nach einem der vorhergehenden Ansprüche, **dadurch gekennzeichnet, dass** das Anschlussstück (40, 60) mindestens ein distales Element (41, 61) und ein proximales Element (42, 62) mit dem konischen Abschnitt (45) aufweist, wobei das distale Element (41, 61) mit dem proximalen Element (42, 62) vorzugsweise mittels einer Schnappverbindung oder eine Schweißverbindung miteinander verbindbar sind.

6. Katheter nach Anspruch 5, **dadurch gekennzeichnet, dass** das distale Element (41, 61) an seinem distalen Ende eine Knickschutz-Einrichtung (52) aufweist.

7. Katheter nach einem der Ansprüche 5 bis 7, **dadurch gekennzeichnet, dass** das Anschlussstück (20, 60) oder das distale Element (41, 61) des Anschlussstücks (40) an der Innenseite seines distalen Endes (21) mit der Außenfläche des ersten Schlauchs (11) verklebt ist.

## Claims

1. Catheter having a catheter shaft (10) comprising a first tube (11) forming a first lumen and a second tube (12) forming a second lumen and disposed at least partially within the first tube (11), the second tube (12) being at least partially welded with its outer surface to the inner surface of the first tube (11),
**characterized in that** the catheter shaft (10) is connected to a connector (20, 40) having at least one conical portion (25, 45) onto which the proximal end of the second tube (12) is slid.

2. Catheter according to claim 1, **characterized in that** the weld (15) extends parallel to the longitudinal axis of the first tube (11) and/or the second tube (12).

3. Catheter according to one of the preceding claims, **characterized in that** the first tube (11) has a laser-active material at least in the region of its inner surface and/or the second tube (12) has a laser-active material at least in the region of its outer surface.

4. Catheter according to any one of the preceding claims, **characterized in that** the first tube (11) comprises a first chemical composition and the second tube (12) comprises a second chemical composition, wherein the second chemical composition is different from the first chemical composition.

5. Catheter according to any one of the preceding claims, **characterized in that** the connector (40, 60) comprises at least one distal element (41, 61) and one proximal element (42, 62) with the conical section (45), wherein the distal element (41, 61) is connectable to the proximal element (42, 62) preferably by means of a snap connection or a weld connection.

6. Catheter according to claim 5, **characterized in that** the distal member (41, 61) has an anti-kink device (52) at its distal end.

7. Catheter according to any one of claims 5 to 7, **characterized in that** the connector (20, 60) or the distal element (41, 61) of the connector (40) is glued to the outer surface of the first tube (11) on the inner side of its distal end (21).

## Revendications

1. Cathéter ayant une tige de cathéter (10) comprenant un premier tube (11) formant une première lumière et un second tube (12) formant une seconde lumière et disposé au moins partiellement à l'intérieur du premier tube (11), dans lequel le second tube (12) est soudé au moins en partie par sa surface extérieure à la surface intérieure du premier tube (11),
**caractérisé en ce que** la tige de cathéter (10) est reliée à une pièce de raccordement (20, 40) qui présente au moins une section conique (25, 45) sur laquelle est enfilée l'extrémité proximale du deuxième tube (12).

2. Cathéter selon la revendication 1, **caractérisé en ce que** la soudure (15) s'étend parallèlement à l'axe longitudinal du premier tube (11) et/ou du deuxième tube (12).

3. Cathéter selon l'une quelconque des revendications précédentes, **caractérisé en ce que** le premier tube (11) comporte un matériau actif au laser au moins au niveau de sa surface interne et/ou le deuxième tube (12) comporte un matériau actif au laser au moins au niveau de sa surface externe.

4. Cathéter selon l'une quelconque des revendications précédentes, **caractérisé en ce que** le premier tube (11) a une première composition chimique et le deuxième tube (12) a une deuxième composition chimique, la deuxième composition chimique étant différente de la première composition chimique.

5. Cathéter selon l'une des revendications précédentes, **caractérisé en ce que** la pièce de raccordement (40, 60) comprend au moins un élément distal (41, 61) et un élément proximal (42, 62) avec la section conique (45), l'élément distal (41, 61) pouvant être relié à l'élément proximal (42, 62), de préférence au moyen d'une liaison par encliquetage ou d'une liaison par soudage.

6. Cathéter selon la revendication 5, **caractérisé en ce que** l'élément distal (41, 61) comporte un dispositif anti-pliure (52) à son extrémité distale.

7. Cathéter selon l'une des revendications 5 à 7, **caractérisé en ce que** l'embout (20, 60) ou l'élément distal (41, 61) de l'embout (40) est collé sur la face interne de son extrémité distale (21) à la surface externe de la première tubulure (11).
